# EUROPEAN PATENT SPECIFICATION

(11) **EP 2 939 553 B1**
(45) Date of publication and mention of the grant of the patent: **28.06.2017**
(21) Application number: 13869760.2
(22) Date of filing: 25.12.2013
(51) Int. Cl.: A24F 47/00, A24B 15/32, A24B 15/42, A24B 15/10, A24B 15/14, A24B 15/18, A24B 15/16

(54) **FLAVOR SOURCE FOR NON-COMBUSTION INHALATION-TYPE TOBACCO PRODUCT, AND NON-COMBUSTION INHALATION-TYPE TOBACCO PRODUCT**
GESCHMACKSQUELLE FÜR EIN NICHTBRENNENDES INHALIERBARES TABAKPRODUKT UND NICHTBRENNENDES INHALIERBARES TABAKPRODUKT
SOURCE DE SAVEUR POUR PRODUIT DE TABAC DU TYPE À INHALATION SANS COMBUSTION, ET PRODUIT DE TABAC DU TYPE À INHALATION SANS COMBUSTION

(30) Priority: 28.12.2012 JP 2012288532
(43) Date of publication of application: 04.11.2015
(73) Proprietor: JAPAN TOBACCO INC., Minato-ku Tokyo 105-8422 (JP)
(72) Inventor: SHINKAWA, Takeshi, Tokyo 130-8603 (JP); MATSUMOTO, Hirofumi, Tokyo 130-8603 (JP); HIGUCHI, Kimitaka, Tokyo 130-8603 (JP); KATAYAMA, Kazuhiko, Tokyo 130-8603 (JP); YAMADA, Manabu, Tokyo 130-8603 (JP)
(74) Representative: Isarpatent
(86) International application number: PCT/JP2013/084629
(87) International publication number: WO 2014/104078

(56) References cited:
- EP-A2- 0 339 658
- WO-A1-2010/095660
- WO-A1-2012/026481
- WO-A1-2012/133289
- WO-A1-2013/111320
- WO-A1-2013/111792
- WO-A1-2013/122054
- CA-A1- 2 807 635
- JP-A- H01 191 674
- JP-A- 2011 509 667
- JP-B2- H 069 497
- US-A1- 2011 290 267

## Description

### [Technical Field]

The present invention relates to a flavor source for a non-burning inhalation type tobacco product that enables inhaling of flavors without burning, and a non-burning inhalation type tobacco product.

### [Background Art]

In recent years, in order to control the occurrence of smoke, a non-burning inhalation type tobacco product that enables inhaling of flavors without burning has been provided (for example, see Patent Literature 1). A method of manufacturing a tobacco compact is disclosed in Patent Literature 1 as a flavor source for a non-burning inhalation type tobacco product. The manufacturing method according to Patent Literature 1 includes a step of mixing a binding agent with a granular tobacco material, and a step of applying pressure to the mixed tobacco material and binding agent. According to the manufacturing method described in Patent Literature 1, it is possible to obtain the tobacco compact formed in a fixed shape and has air permeability, by solidifying the granular tobacco material with the binding agent.

Moreover, in order to comply with the favor of users, a non-burning inhalation type tobacco product containing menthol component has been provided. The non-burning inhalation type tobacco product containing menthol component, for example, is manufactured by adding a menthol solution to the tobacco compact formed in a fixed shape. Moreover, in the non-burning inhalation type tobacco, menthol component must be added to the air that passes through the flavor source as a result of inhalation by the user, without burning the flavor source such as tobacco compact. Thus, in order to deliver a sufficient menthol flavor to the user, the proportion of the weight of menthol component with respect to the weight of the tobacco material in the non-burning inhalation type tobacco must be higher as compared to the proportion of the weight of menthol component with respect to the weight of the tobacco material in a burning inhalation type tobacco, such as a cigarette.

However, if the proportion of the weight of menthol component with respect to the weight of the tobacco material is high, the menthol component once infiltrated into the tobacco compact may be deposited on the surface of the tobacco compact or the inhaling holder. If menthol component is deposited on the surface of the tobacco compact or the inhaling holder, the appearance of the tobacco product may deteriorate, or discomfort may be felt since the deposited menthol component may adhere on to the hands of the user upon holding.

### [Citation List]

### [Patent Literature]

[Patent Literature 1] Japanese Patent Application Publication No. Heisei 06-9497 shredded tobacco leaf pellet composed of tobacco leaf shreds bound together with a nicotine non-absorptive thermoplastic binder while retaining air permeability. The pellet is produced by mixing tobacco leaf shreds and a nicotine-non-absorptive thermoplastic binder, forming the resultant mixture into a desired shape while retaining air permeability, and then subjecting the thus-formed mixture to a heat treatment. Shredded tobacco leaf pellets such obtained may optionally be impregnated with a flavouring, a tobacco extract flavour in an amount as needed (not specified). A cigarette-like snuff is composed of at least one shredded tobacco leaf pellet of the above-described type and an outer envelope surrounding the pellet therein . a non-heating type apparatus for inhaling flavors that has a hollow cylindrical shape. The apparatus comprises: a holder having an air introduction hole at a front end and an inhalation hole at a rear end; and a flavor cartridge running inside the holder from the air introduction hole toward the inhalation hole along the axis of the holder. The flavor cartridge houses a flavor-generating material which contains a granular tobacco material and the flavor-generating material is maintained in a predetermined required state of compression. Document US20110290267 discloses a non-heating flavor inhaler comprising a suction holder including an upstream section extending from a front end thereof to a partition in the suction holder, and a downstream section extending from the front end thereof to a mouth end in the suction holder, the downstream section having a front passage extending along the upstream section, an ambient air intake provided in a circumferential wall of the suction holder to connect the upstream section to the outside, and a pouch for releasing a tobacco flavor, the pouch being located on the boundary between the upstream section and the downstream section and extending along a longitudinal axis of the suction holder.

### [Summary of Invention]

As a result of performing intense experiments and examinations, the present inventors discovered that the deposition amount of menthol component varies depending on the difference in the binding agent. More particularly, it was understood that the deposition of menthol component varies depending on the relationship between the solubility parameter of the binding agent and the solubility parameter of the menthol component.

A flavor source for a non-burning inhalation type tobacco product according to the present invention is made based on the above-knowledge. A first feature is summarized as a flavor source for a non-burning inhalation type tobacco product formed by thermal fusion bonding, comprising: a tobacco material having granular shape, a binding agent that binds the tobacco material, and a menthol component, wherein a proportion of a weight of the menthol component with respect to a weight of the tobacco material is 0.15 or more, and a difference between a solubility parameter of the binding agent and a solubility parameter of menthol component is 2.0 or less.

A second feature is summarized as a non-burning inhalation type tobacco product, comprising: the flavor source for the non-burning inhalation type tobacco product according to the first feature: a holder that contains the flavor source, and a supporting member that supports the flavor source inside the holder and increases an exposed area of the flavor source within the holder beyond a cross-sectional area specified by the inner circumference of the holder.

A third feature is summarized as a non-burning inhalation type tobacco product comprising: the flavor source for the non-burning inhalation type tobacco product according to the first feature: a heat source that heats the flavor source without burning, and having a plate-shape including a pair of main surfaces, and a holder that contains the heat source and the flavor source, wherein the flavor source has a plate-shape including a pair of main surfaces, and the heat source and the flavor source are laminated inside the holder so that one of the main surfaces provided on the heat source and one of the main surfaces provided on the flavor source are facing each other, the flavor source has an inlet that leads air into the flavor source, and an outlet that leads the air out from the flavor source, and the inlet and the outlet are provided on the surfaces other than the main surface facing the heat source among surfaces provided on the flavor source.

A fourth feature is summarized as a non-burning inhalation type tobacco product, comprising: the flavor source for the non-burning inhalation type tobacco product according to the first feature; a heat source that heats the flavor source; and a holder that contains the flavor source and the heat source, wherein the heat source includes a latent heat storage material.

### [Brief Description of Drawings]

[Fig. 1] Fig. 1 is a perspective schematic view of a flavor source for a non-burning inhalation type tobacco product according to a first embodiment.
[Fig. 2] Fig. 2 is a cross-sectional view of an A-A cross-section of the flavor source for the non-burning inhalation type tobacco product illustrated in Fig. 1.
[Fig. 3] Fig. 3 is a cross-sectional view showing a non-burning inhalation type tobacco product according to a second embodiment.
[Fig. 4] Fig. 4 is a cross-sectional view taken along line B-B of Fig. 3.
[Fig. 5] Fig. 5 is a partially fractured perspective view of a non-burning inhalation type tobacco product according to a third embodiment.
[Fig. 6] Fig. 6 is a drawing showing a deformation of a tubular member of the non-burning inhalation type tobacco product of Fig. 5.
[Fig. 7] Fig. 7 is a longitudinal cross-sectional view of the non-burning inhalation type tobacco product after the deformation of the tubular member.
[Fig. 8] Fig. 8 is a perspective view of a non-burning inhalation type tobacco product according to a fourth embodiment.
[Fig. 9] Fig. 9 is a perspective view of the non-burning inhalation type tobacco product according to the fourth embodiment.
[Fig. 10] Fig. 10 is a cross-sectional view in a shorter direction of the non-burning inhalation type tobacco product according to the fourth embodiment.
[Fig. 11] Fig. 11 is a cross-sectional view in a longitudinal direction of the non-burning inhalation type tobacco product according to the fourth embodiment.
[Fig. 12] Fig. 12 is a perspective view of the non-burning inhalation type tobacco product having an open lid according to the fourth embodiment.
[Fig. 13] Fig. 13 is a drawing showing a flow path of air in a case when the flavor source for the non-burning inhalation type tobacco product, and a heat source are inserted in the non-burning inhalation type tobacco product according to the fourth embodiment.

### [Description of Embodiments]

Hereinafter, the embodiments of the present invention will be described with reference to the drawings. In the following drawings, identical or similar components are denoted by identical or similar reference numerals.

Therefore, specific dimensions should be determined with reference to the description below. It is needless to mention that different relationships and ratio of dimensions may be included in different drawings.

### (1) Configuration of flavor source for non-burning inhalation type tobacco product

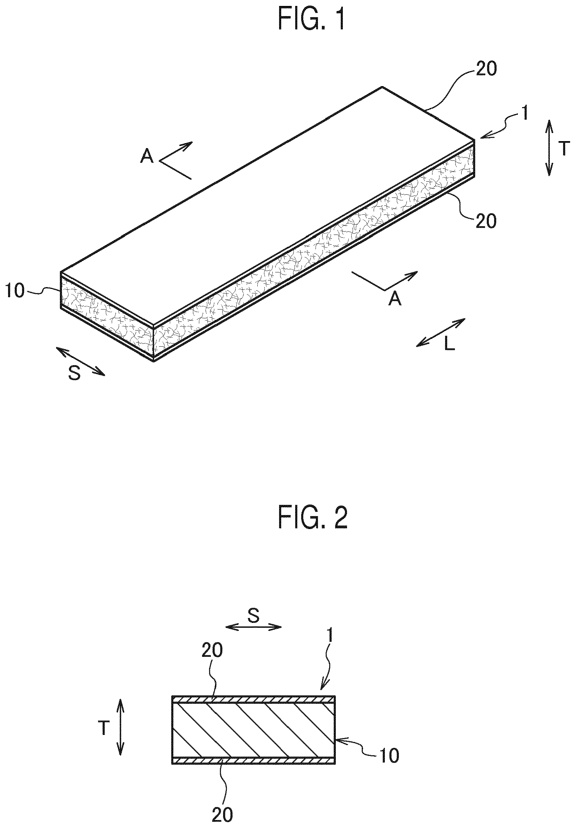

The configuration of a flavor source 1 for a non-burning inhalation type tobacco product according to a first embodiment is described below on the basis of Fig. 1 and Fig. 2. Fig. 1 is a perspective schematic view of the flavor source 1 for the non-burning inhalation type tobacco product according to the first embodiment. Fig. 2 is a cross-sectional view of an A-A cross-section of the flavor source 1 for the non-burning inhalation type tobacco product shown in Fig. 1.

The flavor source 1 for the non-burning inhalation type tobacco product is formed in a plate-shape, and has an approximately rectangular parallelepiped shape having a longitudinal direction L, a shorter direction S, and a thickness direction T. The flavor source 1 for the non-burning inhalation type tobacco product has a tobacco compact 10 including a tobacco material, and a pair of nonwoven cloths 20 arranged to sandwich the tobacco compact 10.

The tobacco compact 10 has a tobacco material, a binding agent, and menthol component. The tobacco material has a granular shape prior to a forming process. The tobacco compact 10 is realized by binding the granular tobacco material using the binding agent. The binding agent will be described later in detail.

The proportion of the binding agent with respect to the weight of the tobacco material is 0.5 or more and 3.0 or less. If the proportion of the binding agent with respect to the weight of the tobacco material is less than 0.5, the tobacco material may not be solidified properly, and there is possibility that a sufficient amount of the menthol component may not be held. If the proportion of the binding agent with respect to the weight of the tobacco material is more than 3.0, the flavor source may become too thick, and the air permeability may decline.

The menthol component is added to the tobacco material formed in the plate-shape. It is possible to use various methods for adding the menthol component to the tobacco compact, for example, it is possible to directly spray a menthol solution prepared by dissolving menthol component in a well-known solvent, such as ethanol, on the tobacco compact. Although not shown in the figures, menthol component is deposited not only on the surface of the tobacco compact 10, but also infiltrates inside.

The proportion of the weight of the menthol component with respect to the weight of the tobacco material is 0.15 or more. Since the flavor source 1 for the non-burning inhalation type tobacco product according to the present embodiment provides the menthol component to the user without burning, the proportion of the weight of menthol component with respect to the weight of the tobacco material is higher than a flavor source for a general non-burning inhalation type tobacco product. For example, the proportion of the weight of menthol component with respect to the weight of the tobacco material in a burning inhalation type tobacco is approximately 0.01. This menthol component weight is the weight of menthol component that remains in the flavor source 1 for the non-burning inhalation type tobacco product after going through the manufacturing process described later.

The tobacco compact 10 has air permeability, and is configured such that the air added with the flavor of the tobacco material passes through the tobacco compact 10 when the air is led in as a result of inhalation by the user. The nonwoven cloths 20 have air permeability, and is arranged to cover a pair of opposite surfaces of the tobacco compact. The air permeability may be configured such that the inhaled air passes through the inside of the flavor source 1 for the tobacco product, and it is possible to deliver the tobacco material and flavor components of menthol component to the user.

As for the usage method of the flavor source 1 for the non-burning inhalation type tobacco product thus configured, the user may inhale the flavor components by using an end of the longitudinal direction L of the flavor source 1 for the non-burning inhalation type tobacco product as a mouthpiece, or the flavor source 1 may be contained in a holder or the like shown in a second embodiment through a fourth embodiment and the user may inhale the air via the mouthpiece provided in the holder. More preferably, the flavor source 1 is configured to enable inhaling via the holder. In addition, the flavor source 1 for the non-burning inhalation type tobacco product may be configured to provide flavor components to the user without heating, or may be configured to provide flavor components to the user by a heat source that generates heat by electric heat, chemical reaction heat, or burning of a carbonaceous material, without burning of the flavor source. For example, by using a latent heat storage material that makes use of an exoergic reaction based on latent heat (heat of crystallization or heat of solidification) as the heat source, it is possible to repeatedly use the heat source, and it is possible to realize a flavor inhaler having a simple configuration.

Since the flavor source 1 for the non-burning inhalation type tobacco product according to the present embodiment includes the nonwoven cloths 20 having air permeability, it is possible to protect the surface of the tobacco compact 10 by the nonwoven cloth 20. Thus, it is possible to prevent the tobacco material from falling out and being separated of the tobacco compact 10 while securing air permeability. Moreover, it is possible to favorably use not only a nonwoven cloth but also any material which has air permeability and which is capable of retaining a tobacco compact 10.

### (2) Method of manufacturing flavor source for non-burning inhalation type tobacco product

Next, an example of a method of manufacturing the flavor source 1 for the non-burning inhalation type tobacco product will be described. In a first step, a granular tobacco material and a binding agent are mixed. In a second step, the tobacco material and the binding agent mixed in the first step are sandwiched by the nonwoven cloths 20. In a third step, the product is formed in a fixed shape by thermal fusion bonding. As a result of the thermal fusion bonding, it is possible to form the tobacco compact 10 and the nonwoven cloth 20 as an integrated body. In a fourth step, a menthol solution prepared by dissolving menthol component is sprayed on the formed product formed in the third step, or the formed product is immersed in a menthol solution prepared by dissolving menthol component so that menthol component infiltrates into the entire formed product.

Moreover, it is possible to make the formed product thus obtained to have a predetermined size and shape by a method such as cutting. In addition, thermal fusion bonding of the nonwoven cloth present on both surfaces may be performed on the peripheral portions of the formed product. As a result, it is possible to prevent the loss of the tobacco material from the peripheral portions of the formed product. Here, for example, cutting may be performed while providing heat to enable fusion of the nonwoven cloth present on both surfaces using a method such as thermal cutting. Thus, it is possible to perform thermal fusion bonding of the peripheral portions simultaneously of cutting, enabling simplification of the manufacturing process.

According to the manufacturing method, it is possible to obtain the flavor source 1 for the non-burning inhalation type tobacco product in which menthol component has been added to the entire flavor source 1 for the non-burning inhalation type tobacco product. As a result of thermal fusion bonding, it is possible to fuse the nonwoven cloths while forming the tobacco compact containing the tobacco material or the like, and it is thus possible to simplify the manufacturing process. It must be noted that the method of manufacturing the flavor source for the non-burning inhalation type tobacco product according to the present embodiment is not limited to the above-described method, and a method that enables bonding of the tobacco material through thermal fusion bonding via a binding agent may also be used.

Moreover, the tobacco material may include an additive including at least one of a carbonate or a hydrogen carbonate. With the help of the additive including at least one of a carbonate or a hydrogen carbonate, a pH regulation process may be performed in the course of manufacturing the tobacco compact. In the pH regulation process, the pH of the tobacco material is increased up to an alkaline pH, for example, up to 8.0 to 11.0, more preferably, up to 9.0 to 10.0. Moreover, by adding the pH regulator as an aqueous solution, it is possible to simultaneously regulate the percentage of water content in the tobacco material and the pH, and it is possible to shorten the processing time.

### (3) Binding agent

Next, the binding agent included in the tobacco compact 10 will be described in detail. The binding agent used in the present invention is made of a thermoplastic resin or a heat-hardening resin, and the difference between the solubility parameter of the binding agent and the solubility parameter of menthol component is 2.0 or less. Specifically, the binding agent is selected from a group consisting of an ethylene vinylacetate copolymer (EVA), a polyvinyl alcohol (PVA), polyurethane, polyamide, and a combination of these.

The solubility parameter is a material-specific parameter that expresses the solubility, and hydrophilic/hydrophobic property of a material. The solubility parameter is defined as the square root of the evaporation heat (cal/cm³)^{1/2} necessary for the evaporation of 1 cm³ of liquid, for example, the solubility parameter is calculated by the Hildebrand method and the Fedors method, etc. Moreover, in the present embodiment, a value converted to the SI System of Units (MPa ^{1/2}) is described on the basis of the following formula: 2.046 (MPa ^{1/2}) = 1.00 (cal/cm³)^{1/2}

Specifically, the solubility parameter of menthol component in the present embodiment is 18.8. In the present embodiment, the solubility parameter of the ethylene vinylacetate copolymer (EVA) is 18.0 to 19.2, the solubility parameter of the polyvinyl alcohol (PVA) is 19.2, the solubility parameter of the polyurethane is 20.5, and the solubility parameter of the polyamide is 19.4.

Note that in the present embodiment, a group consisting of an ethylene copolymer such as an ethylene vinylacetate copolymer (EVA), a polyvinyl alcohol (PVA), polyurethane, polyamide, and a combination of these was cited as the binding agent, however, the binding agent according to the present invention is not limited to this, and it is possible to use a binding agent for which the difference between the solubility parameter of the binding agent and the solubility parameter of menthol component is 2.0 or less.

For example, 1:EMMA: Ethylene methyl methacrylate copolymer ("Acryft CM4013" manufactured by Sumitomo Chemical Co., Ltd.)having solubility parameter of 18.6 to 19.2, 2: EEA: Ethylene ethyl acrylate copolymer ("Evaflex EEA-A709" manufactured by DuPont-Mitsui Chemical Co., Ltd.)having solubility parameter of 19.2, 3: EMAGMA: Ethylene methacrylate glycidyl methacrylate copolymer ("Bondfast 7L" manufactured by Sumitomo Chemical Co., Ltd.) having solubility parameter of 18.4 to 19.4, 4: EEAMAH: Ethylene-ethyl acrylate maleic anhydride copolymer ("Bondine AX-8390" manufactured by Sumitomo Chemical Co., Ltd.) having solubility parameter of 18.4 to 19.4, 5: EVA: Ethylene vinylacetate copolymer ("Evaflex EV150" manufactured by DuPont-Mitsui Chemical Co., Ltd.) having solubility parameter of 18.0 to 19.2 may be used as the ethylene copolymer, the above-mentioned various ethylene copolymers may be added and mixed in various proportions to a thermoplastic elastomer, and then pelleted with a pelletizer for use.

A denatured PVA denatured by a method known per se may be used as the polyvinyl alcohol, and a copolymer with PVA as the main constituent may also be used as long as the objective of the present invention.

The menthol component is a volatile flavor, and evaporates readily from the tobacco compact as a result of an increase in the storage time and the rise in temperature during the storage period. Moreover, generally, the flavor source 1 for the non-burning inhalation type tobacco product is encapsulated in a package, such as a box. Thus, when the flavor source 1 for the non-burning inhalation type tobacco product includes a large amount of menthol component, the evaporated menthol component easily reaches the saturated concentration in the package, and therefore, due to the effect of the continuous change in temperature, the evaporated menthol component easily is deposited on the surface of the flavor source 1 for the non-burning inhalation type tobacco product in the package. However, since the binding agent of the present embodiment has a difference of 2.0 or less between the solubility parameter of the binding agent and the solubility parameter of menthol component, the affinity between menthol component and the binding agent is high, and therefore, at the time of storage, it is possible to prevent the evaporation of menthol component from the tobacco compact during storage. This makes it difficult for menthol component to be deposited within the package. Therefore, it is possible to ensure that the flavor source 1 for the non-burning inhalation type tobacco product is made to carry a sufficient amount of menthol component, thus making it possible to obtain the flavor source for the non-burning inhalation type tobacco product capable of providing the menthol component to the user in a stable manner.

Moreover, it is possible to more favorably use EVA as a binding agent. Since EVA has a low fusion point, it offers excellent thermal fusion bonding. Thus, by using EVA, it is possible to improve the manufacturing efficiency.

### (4) Non-burning inhalation type tobacco product

Next, a non-burning inhalation type tobacco product having the above-described flavor source for the non-burning inhalation type tobacco product will be described.

### (4.1) Non-burning inhalation type tobacco product according to a second embodiment

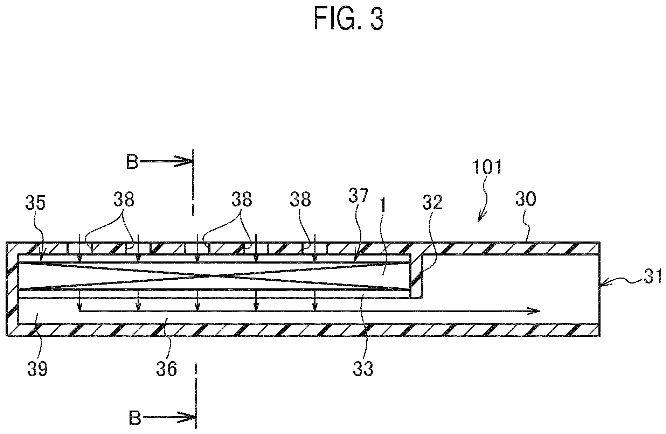

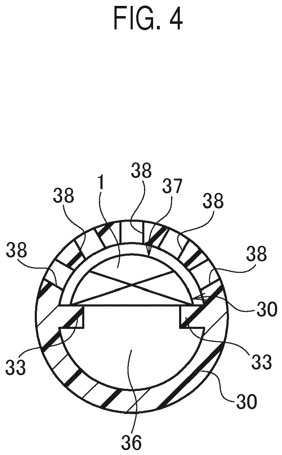

Fig. 3 and Fig. 4 are drawings showing the non-burning inhalation type tobacco product 101 according to the second embodiment. Fig. 3 is a cross-sectional view showing the non-burning inhalation type tobacco product according to the second embodiment. Fig. 4 is a cross-sectional view taken along line B-B of Fig. 1. The non-burning inhalation type tobacco product 101 according to the second embodiment includes a flavor source 1 for the non-burning inhalation type tobacco product, a holder that contains the flavor source for the non-burning inhalation type tobacco product, and a supporting member is provided which supports the flavor source 1 inside the holder and increases the exposed area of the flavor source within the holder beyond the cross-sectional area specified by the inner circumference of the holder.

The supporting member is configured to support the flavor source inside the holder, and to partition the air flow path into an upstream portion facing at least either one of the upstream portion and the downstream portion at a front end side and a downstream portion at a mouthpiece end side by the flavor source for the non-burning inhalation type tobacco product, and increase the exposed area of the flavor source that faces at least either one of the upstream portion and the downstream portion beyond the cross-sectional area specified by the inner circumference of the holder.

The non-burning inhalation type tobacco product 101 has an inhaling holder 30 as a holder made of a synthetic resin. The holder has a longitudinal axial line, a front end, and a rear end as a mouthpiece end, and defines an air flow path through which the external air led in from the front end side is led up to the earlier-mentioned mouthpiece end. The inhaling holder 30 has a hollow cylindrical shape, includes an open proximal end and a closed front end wall, and the proximal end of the inhaling holder 30 forms a mouthpiece end 31. A semi-circular partition wall 32 is formed inside the inhaling holder 30, and the partition wall 32 is positioned toward the side of the mouthpiece end 31 from the center when seen in the direction of the axial line of the inhaling holder 30. The partition wall 32 protrudes out from the inner circumference of the inhaling holder 30, and blocks the region corresponding to approximately half of the cross-section. In addition, a pair of ribs 33 are formed on the inner circumference of the inhaling holder 30. The ribs 33 are arranged so as to sandwich the longitudinal axial line of the inhaling holder 30, and extend parallel to the longitudinal axial line of the inhaling holder 30 from the inner edge of the partition wall 32 up to the front end wall of the inhaling holder 30.

The flavor source 1 for the non-burning inhalation type tobacco product is arranged on the earlier-mentioned ribs 33. In particular, the flavor source 1 for the non-burning inhalation type tobacco product is formed along the longitudinal axial line of the inhaling holder 30, extends from the front end wall of the inhaling holder 30 up to the partition wall 32, and has a width almost equal to the inner diameter of the inhaling holder 30. The flavor source 1 for the non-burning inhalation type tobacco product and the partition wall 32 divide the inside of the inhaling holder 30 into the upstream portion 35 and the downstream portion 36. The upstream portion 35 is surrounded by the front end wall of the inhaling holder 30, the partition wall 32, the flavor source 1 for the non-burning inhalation type tobacco product, and the inner circumference of the inhaling holder 30 from the front end wall across the partition wall 32, and the downstream portion 36 extends from the front end wall of the inhaling holder 30 up to the mouthpiece end 31. In particular, the downstream portion 36 extends along the upstream portion 35, and includes a front flow path having a semi-circular cross-section, and a rear flow path having a circular cross section extending from the front flow path up to the mouthpiece end 31. Therefore, the exposed area of the flavor source 1 for the non-burning inhalation type tobacco product that faces the upstream portion 35 is sufficiently wider than the cross-section area specified by the inner diameter of the inhaling holder 30.

In addition, the flavor source 1 for the non-burning inhalation type tobacco product is supported by a pair of ribs 33 at both side edges, and is secured by the ribs 33, and is thus immovable in the upstream portion 35. In other words, the ribs 33 function as the supporting member of the flavor source 1 for the non-burning inhalation type tobacco product. Therefore, when the flavor source 1 for the non-burning inhalation type tobacco product is arranged in the upstream portion 35, then as seen clearly in Fig. 3 and Fig. 4, it must be noted that a predetermined space 37 is secured from the front end wall of the inhaling holder 30 across the partition wall 32 between the circumferential wall of the inhaling holder 30 and the flavor source 1 for the non-burning inhalation type tobacco product.

In addition, external air inlet openings 38 are formed in a distributed manner on the circumferential wall of the inhaling holder 30 in which the upstream portion 35 is formed, and the external air inlet openings 38 link the upstream portion 35 with the outside of the inhaling holder 30. Therefore, the inhaling holder 30 has an air flow path 39 extending from the external air inlet openings 38 up to the mouthpiece end 31 through the upstream portion 35 and the downstream portion 36.

According to the non-burning inhalation type tobacco product 101 described above, when the user inhales air from the mouthpiece end 31 of the inhaling holder 30, then as illustrated by the arrows in Fig. 3, the air from outside enters from the external air inlet openings 38 of the inhaling holder 30 to the upstream portion 35, that is, inside the space 37 described earlier, and thereafter, the entered air passes through the inside of the flavor source 1 for the non-burning inhalation type tobacco product from the entire region of the space 37 in the radial direction inhaling holder 30, and reaches the downstream portion 36. In other words, the air passes through the inside of the flavor source 1 for the non-burning inhalation type tobacco product along the direction of the longitudinal axial line of the inhaling holder 30, uniformly throughout the entire region. At this time, as a result of a favorable contact with the granular body inside the flavor source 1 for the non-burning inhalation type tobacco product, the air is capable of including the tobacco flavor components released from the granular body. Following this, the air inside the downstream portion 36 is led to the mouthpiece end 31, and reaches inside the mouth cavity of the user. Thus, since the tobacco flavor components are included in the air inhaled by the user, the user is capable of effectively tasting the tobacco flavor. Moreover, by adopting the flavor source 1 for the non-burning inhalation type tobacco product according to the present invention as the flavor source, it is possible to ensure that the main body is made smaller in size, as compared to a case in which a pouch or the like packed with granular tobacco body is adopted as the flavor source, for example.

### (4.2) Non-burning inhalation type tobacco product according to a third embodiment

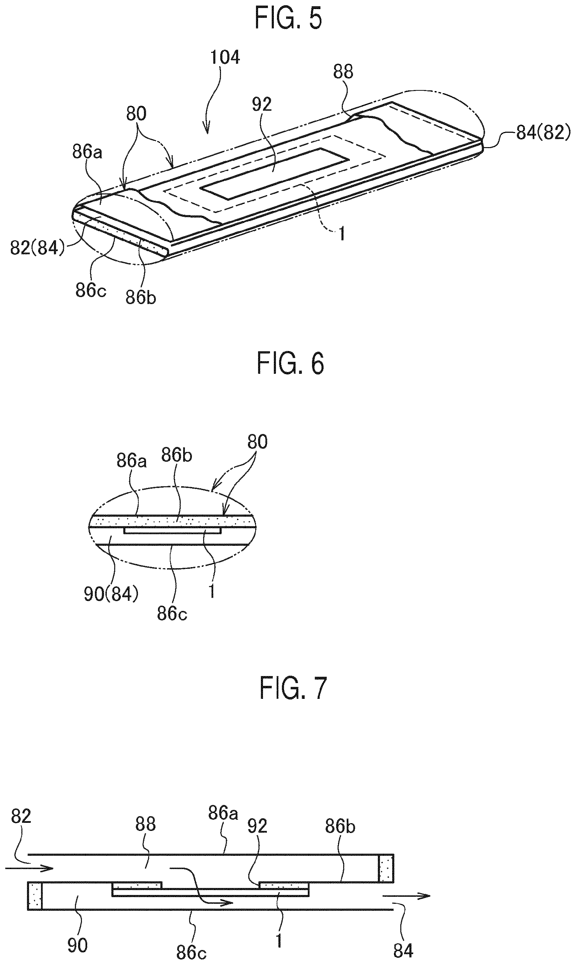

Fig. 5 through Fig. 7 are drawings showing a non-burning inhalation type tobacco product 104 according to the third embodiment. Fig. 5 is a partially fractured perspective view of the non-burning inhalation type tobacco product 104 according to the third embodiment. Fig. 6 is a drawing showing a deformation of a tubular member of the non-burning inhalation type tobacco product 104 of Fig. 5. Fig. 7 is a longitudinal cross-sectional view of the non-burning inhalation type tobacco product 104 after the deformation of the tubular member.

The non-burning inhalation type tobacco product 104 includes a flavor source for the non-burning inhalation type tobacco product, a holder that contains the flavor source for the non-burning inhalation type tobacco product, and a supporting member which supports the flavor source inside the holder and increases the exposed area of the flavor source within the holder beyond the cross-sectional area specified by the inner circumference of the holder. The holder is a tubular member formed by stacking at least three sheets having flexibility. The tubular member includes a first air flow path that opens at one end of the tubular member and is formed between a central sheet positioned in the center, and a first sheet, which is one of the remaining sheets; a second air flow path that opens at the other end of the tubular member and is formed between the central sheet and a second sheet, which is one of the earlier-mentioned remaining sheets other than the first sheet; and an aperture communicates the first and the second air flow paths and is formed in the central sheet. The flavor source for the non-burning inhalation type tobacco product is arranged to cover the aperture on the central sheet.

The non-burning inhalation type tobacco product 104 includes a tubular member 80 as the holder. The tubular member 80 is compressed, and includes openings 82 and 84 at both ends. The tubular member 80 is formed by stacking three rectangular sheets 86a, 86b, and 86c, and each of these sheets 86a, 86b, and 86c has flexibility. As seen in Fig. 5, the sheet 86a positioned on the upper side and the central sheet 86b are used to form the first air flow path 88 that opens to the external air through the opening 82, and the first air flow path 88 has a circumference sealed with respect to the external air either through the sheet itself or by an adhesive, excluding the opening 82.

The sheet 86b and the sheet 86c positioned on the lower side are used to form the second air flow path 90 that opens to the external air through the opening 84, and the second air flow path 90 too has a circumference sealed with respect to the external air either through the sheet itself or by an adhesive, excluding the opening 84.

An aperture 92 is formed in the center of the central sheet 86b, and the aperture 92 extends in the longitudinal direction of the sheet 86b, for example.

The plate-shaped flavor source 1 for the non-burning inhalation type tobacco product is pasted on the surface of the sheet 86b at the side of the second air flow path 90. An adhesive may be used for pasting, and if the outer surface of the flavor source 1 for the non-burning inhalation type tobacco product is formed by a material having the heat seal property, it is also possible to use the thermal fusion bonding of the material. The flavor source 1 for the non-burning inhalation type tobacco product is larger than the aperture 92, and blocks the aperture 92. The flavor source 1 for the non-burning inhalation type tobacco has air permeability, and at the same time, includes a flavor generating material therein. The flavor generating material releases the flavor components to the first and the second air flow path 88 and 90 without being heated.

According to the non-burning inhalation type tobacco product 104 described above, the user first pinches both edges of the tubular member 80 with a finger to compress the tubular member 80 in the width direction. As a result of the compression, the sheets 86a and 86c are pulled apart from each other, because of which as shown by the two-dot chain line in Fig. 5 and Fig. 6, the tubular member 80 is formed in a cylindrical shape, and both the first air flow path 88 having the opening 82, and the second air flow path 90 having the opening 84 secure a large flow path cross-sectional area. In other words, the central sheet 86b functions as a supporting member that supports the flavor source in the holder, and increases the exposed area of the flavor source within the holder beyond the cross-sectional area specified by the inner circumference of the holder.

In this state, when the user takes one end of the tubular member 80 at the side of the opening 84 in his/her mouth as a mouthpiece, and inhales through the opening 84, then as shown by the arrows in Fig. 7, the flow of air is generated from the opening 82 in the tubular member 80, toward the first air flow path 88, the aperture 92, the flavor source 1 for the non-burning inhalation type tobacco product, the second air flow path 90, and the opening 84. Since the flavor components released from the flavor source 1 for the non-burning inhalation type tobacco product are included in the flow of the air, the user is capable of tasting the flavor components.

On the other hand, if the user inhales the air by taking the other end of the tubular member 80 at the side of the opening 82 in his/her mouth as the mouthpiece, the other end becomes the mouthpiece, and similarly, it is possible to taste the flavor components. In such a case, the flow of air inside the tubular member 80 becomes reversed as compared to the case described earlier.

Since the aperture 92 is arranged inside the tubular member 80, it is not exposed to the outside, and therefore, when inhaling the flavor components, the aperture 92 is blocked by the user's fingers, and the inhalation of flavor components is not obstructed.

### (4.3) Non-burning inhalation type tobacco product according to a fourth embodiment

### (4.3.1) Configuration of flavor source for non-burning inhalation type tobacco product

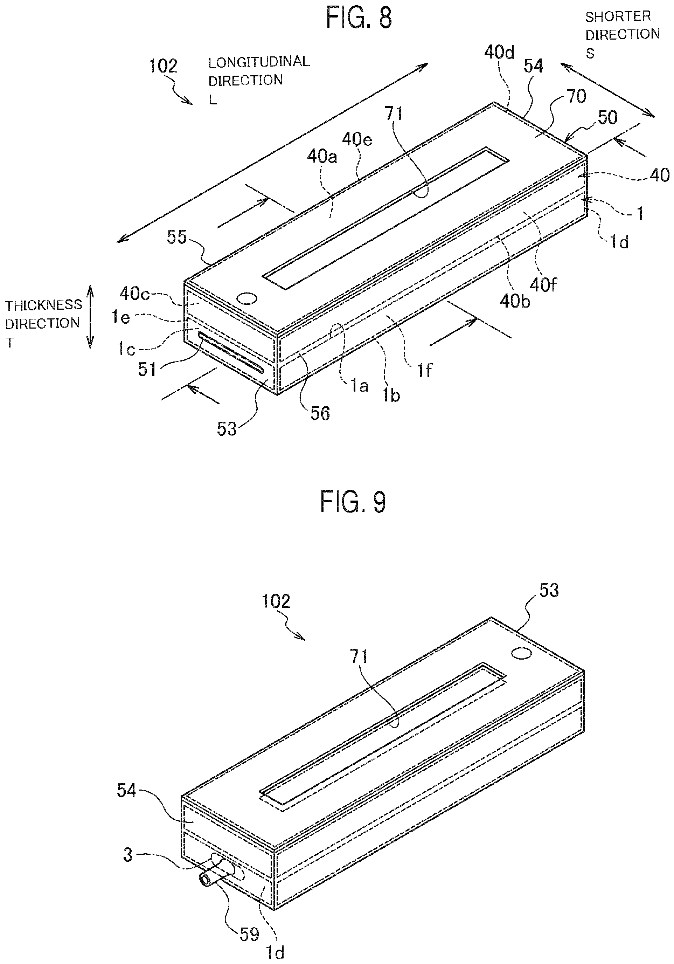

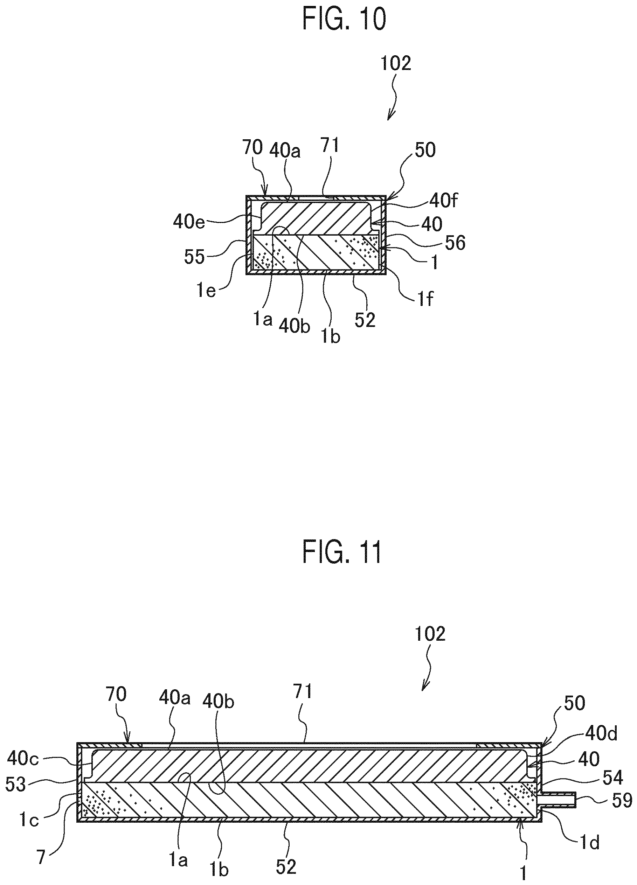

Fig. 8 and Fig. 9 are drawings showing a non-burning inhalation type tobacco product 102 according to the fourth embodiment. Fig. 8 and Fig. 9 are perspective views of the non-burning inhalation type tobacco product 102 according to the fourth embodiment. Fig. 10 is a cross-sectional view in a shorter direction of the non-burning inhalation type tobacco product 102 according to the fourth embodiment. Fig. 11 is a cross-sectional view in a longitudinal direction of the non-burning inhalation type tobacco product 102 according to the fourth embodiment.

As shown in Fig. 8 and Fig. 9, the non-burning inhalation type tobacco product 102 includes a flavor source 1 for the non-burning inhalation type tobacco product, a heat source 40, a holder 50, a lid 70, and a mouthpiece 59. The flavor source 1 for the non-burning inhalation type tobacco product and the heat source 40 are laminated inside the holder 50, and are contained inside the holder 50. The non-burning inhalation type tobacco product 102 has an approximately rectangular parallelepiped shape including a longitudinal direction L, a shorter direction S, and a thickness direction T. The longitudinal direction L, for example, is a direction in which the air passing through the flavor source 1 for the non-burning inhalation type tobacco product flows. The shorter direction S is a direction approximately orthogonal to the longitudinal direction L and the thickness direction T. The thickness direction T is a direction in which the flavor source 1 for the non-burning inhalation type tobacco product, and the heat source 40 are laminated.

Here, the size of the non-burning inhalation type tobacco product 102 in the shorter direction S is smaller than the size of the non-burning inhalation type tobacco product 102 in the longitudinal direction L. Moreover, the size of the non-burning inhalation type tobacco product 102 in the thickness direction T is smaller than the size of the non-burning inhalation type tobacco product 102 in the shorter direction S.

As shown in Fig. 8 and Fig. 9, the flavor source 1 for the non-burning inhalation type tobacco product has the widest area and a plate shape (an approximately rectangular parallelepiped shape) including a pair of main surfaces 1a and 1b defined by the longitudinal direction L and the shorter direction S, a pair of side surfaces 1c and 1d defined by the shorter direction S and the thickness direction T, and a pair of side surfaces 1e and 1f defined by the longitudinal direction L and the thickness direction T. The pair of main surfaces 1a and 1b defined by the shorter direction S and the longitudinal direction L orthogonal to each other have a rectangular shape.

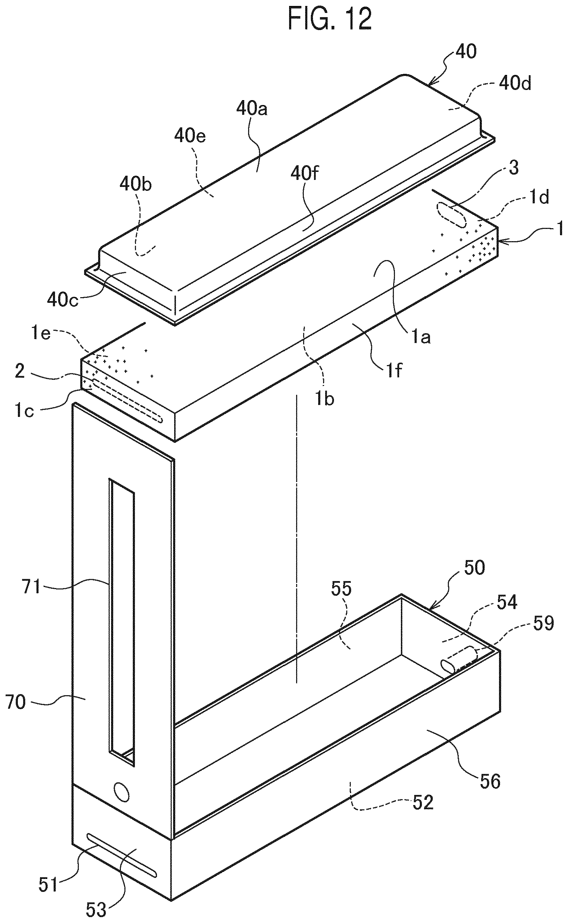

As shown in Fig. 12, the side surface 1c of the flavor source 1 for the non-burning inhalation type tobacco product has an inlet 2 for leading in air. The inlet 2 is a region exposed to an inlet opening 51 of the holder 50 described later.

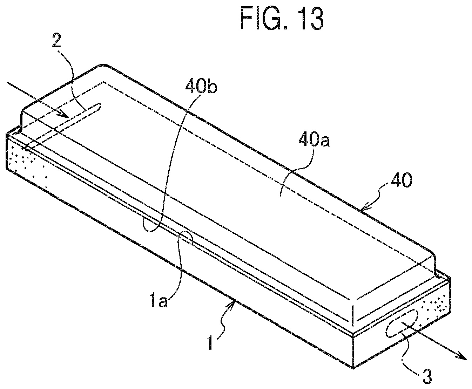

As shown in Fig. 13, the side surface 1d of the flavor source 1 for the non-burning inhalation type tobacco product has an outlet 3 for leading out air. The outlet 3 is a region exposed to a mouthpiece 59 described later. The inlet 2 and the outlet 3 will be described in detail later.

The air led in from the inlet 2 of the flavor source 1 for the non-burning inhalation type tobacco product passes through the inside of the flavor source 1 for the non-burning inhalation type tobacco product, and is led out from the outlet 3. A flavor is added to the air led in from the inlet 2 while passing through the flavor source 1 for the non-burning inhalation type tobacco product.

As shown in Fig. 8 and Fig. 9, the heat source 40 is formed in a plate shape configured by a pair of main surfaces 40a and 40b having the widest area, a pair of side surfaces 40c and 40d along the shorter direction, and a pair of side surfaces 40e and 40f along the longitudinal direction L.

In order to heat the flavor source 1 for the non-burning inhalation type tobacco product, the heat source 40 generates a heat of 60°C or more. Particularly, it is preferable that the heat source 40 generates a heat of 90°C or more. It is possible to adopt electric heat and various types of heat of chemical reactions as the mode of generation of heat by the heat source 40. Chemical reactions that make use of the oxidation reaction heat using the oxygen in the atmosphere, as well as the latent heat are well-known as the chemical reactions accompanying the generation of heat. In the fourth embodiment, it is preferable to use the oxidation reaction heat of iron powder as the oxidation reaction heat. More particularly, the heat source 40 is configured by iron powder and an outer bag that stores the iron powder. The outer bag, for example, is configured by a nonwoven cloth having apertures that are smaller than the grain size of the iron powder.

In such cases, it is preferable to secure air permeability of the heat source 40 in a state when the heat source 40 is stored in the holder 50. As described later, in the fourth embodiment, since the lid 70 has an opening 71, the air is supplied from the opening 71 to the main surface 40a of the heat source 40. On the other hand, the heat source 40 is preferably formed from a member such that the air supplied from the main surface 40a of the heat source 40 is not lead out to the flavor source 1 for the non-burning inhalation type tobacco product from the main surface 40b of the heat source 40 facing the flavor source 1 for the non-burning inhalation type tobacco product. A member that does not have air permeability may be pasted on the main surface 40b of the heat source 40.

If the oxidation reaction heat of iron powder is used as the oxidation reaction heat, the heat source 40 preferably includes iron powder, activated carbon, water, and sodium chloride as the heating element. If the total of the iron powder, activated carbon, water, and sodium chloride in the heating element is assumed to be 100 wt% (weight percent), the iron powder is preferably selected in the range of 30 to 60 wt% (weight percent). Similarly, the activated carbon is preferably selected in the range of 10 to 50 wt% (weight percent). Similarly, water is preferably selected in the range of 10 to 30 wt% (weight percent). Moreover, sodium chloride is preferably selected in the range of 0.5 to 7 wt% (weight percent).

For example, when activated carbon having a specific surface area of 1700 m²/g is used, the weight percent of the iron powder, activated carbon, water, and sodium chloride is preferably 46:30:20:4 with the total of the iron powder, activated carbon, water, and sodium chloride being 100 wt% (weight percent). The total weight of the iron powder, activated carbon, water, and sodium chloride in the heat source 40 is preferably from 0.5 to 1.5 g.

As shown in Fig. 8 and Fig. 9, the holder 50 has a box shape that contains the flavor source 1 for the non-burning inhalation type tobacco product and the heat source 40. Specifically, the holder 50 has a plate-shaped bottom panel 52, a pair of plate-shaped wall panels 53 and 54 that rise up from near the shorter direction S of the bottom panel 52, and a pair of plate-shaped wall panels 55 and 56 that rise up from near the longitudinal direction L of the bottom panel 52.

As shown in Fig. 8, the wall panel 53 of the holder 50 has an inlet opening 51 for leading in air. For example, the inlet opening 51 is an opening having a line shape. The inlet opening 51 may be configured by a plurality of dot-shaped openings.

As shown in Fig. 9, a mouthpiece 59 is provided on the wall panel 54 of the holder 50 for the user to inhale the flavor.

As shown in Fig. 8 and Fig. 9, the lid 70 is formed on the opposite side of the bottom panel 52 of the holder 50. The lid 70 functions as a lid of the holder 50 and has an opening 71. The opening 71 is formed to enable securing sufficient contact area between the heat source 40 and the air.

The mouthpiece 59 leads to the inner side of the holder 50, and has a cylindrical shape. The mouthpiece 59 is formed in the center of the wall panel 54 in the shorter direction S.

As shown in Fig. 10 and Fig. 11, the holder 50 has a holding structure for holding the flavor source 1 for the non-burning inhalation type tobacco product, and the heat source 40. As shown in Fig. 10, the flavor source 1 for the non-burning inhalation type tobacco product, and the heat source 40 are laminated inside the holder 50 so that the main surface 40b of the heat source 40 and the main surface 1a of the flavor source 1 for the non-burning inhalation type tobacco product are facing each other. The main surface 40a of the heat source 40 and the lid 70 are close to each other, and are stored in the holder 50 so that the air is supplied from the main surface 40a of the heat source 40 via the opening 71 of the lid 70. The flavor source 1 for the non-burning inhalation type tobacco product is stored in the holder 50 so that the main surface 1a of the flavor source 1 for the non-burning inhalation type tobacco product and the bottom surface 52 of the holder 50 are in contact. That is, the thickness obtained by laminating the flavor source 1 for the non-burning inhalation type tobacco product and the heat source 40 in the thickness direction T is almost same as the thickness of the holder 50. Furthermore, the side surface 1e of the flavor source 1 for the non-burning inhalation type tobacco product and the side surface 40e of the heat source 40 are close to the wall panel 55 of the holder 50. The side surface 1f of the flavor source 1 for the non-burning inhalation type tobacco product and the side surface 40f of the heat source 40 are close to the wall panel 56 of the holder 50. That is, the width of the flavor source 1 for the non-burning inhalation type tobacco product and the heat source 40 in the shorter direction S, and the width of the holder 50 are almost the same.

As shown in Fig. 11, the flavor source 1 for the non-burning inhalation type tobacco product is stored in the holder 50 so that the side surface 1c of the flavor source for the non-burning inhalation type tobacco product and the inlet opening 51 are facing each other, and the side surface 1d of the flavor source 1 for the non-burning inhalation type tobacco product and the mouthpiece 59 are facing each other. Furthermore, the side surface 1c of the flavor source 1 for the non-burning inhalation type tobacco product and the side surface 40c of the heat source 40 are close to the wall panel 53 of the holder 50. The side surface 1d of the flavor source 1 for the non-burning inhalation type tobacco product and the side surface 40d of the heat source 40 are close to the wall panel 54 of the holder 50. That is, the length of the flavor source 1 for the non-burning inhalation type tobacco product and the heat source 40 in the longitudinal direction L, and the length of the holder 50 are almost the same.

### (4.3.2) Inlet and outlet

Next, the inlet 2 and the outlet 3 of the flavor source 1 for the non-burning inhalation type tobacco product will be described on the basis of Fig. 12. Fig. 8 is a perspective view of the non-burning inhalation type tobacco product 102 having an opened lid 70 according to the fourth embodiment.

The inlet 2 is a region that leads the air flew in from the inlet opening 51 of the holder 50 to the flavor source 1 for the non-burning inhalation type tobacco product and that faces the inlet opening 51 of the holder 50. The outlet 3 is a region that leads out the air passing through the inside of the flavor source 1 for the non-burning inhalation type tobacco product and that faces the mouthpiece 59 of the holder 50 as shown in Fig. 8 in the fourth embodiment. That is, out of the surfaces provided on the flavor source 1 for the non-burning inhalation type tobacco product, the inlet 2 and the outlet 3 are provided on the surfaces other than the main surfaces 1a and 1b facing the heat source 40.

### (4.3.3) Flow path of air in non-burning inhalation type tobacco product

Next, the flow path of air in the non-burning inhalation type tobacco product 102 will be described on the basis of Fig. 13. Fig. 13 is a drawing showing a flow path of air in the flavor source 1 for the non-burning inhalation type tobacco product and the heat source 40, in a case when the flavor source 1 for the non-burning inhalation type tobacco product, and the heat source 40 are inserted in the holder 50 of the non-burning inhalation type tobacco product 102 according to the fourth embodiment.

As shown in Fig. 13, the flavor source 1 for the non-burning inhalation type tobacco product and the heat source 40 are laminated and stored inside the holder 50 so that one main surface 40b of the heat source 40 and one main surface 1a of the flavor source 1 for the non-burning inhalation type tobacco product are facing each other, and the entire surface of the main surface 1a of the flavor source 1 for the non-burning inhalation type tobacco product is heated via the entire surface of the main surface 40b of the heat source 40. At this time, upon being inhaled by the user, the air led in from the inlet 2 of the flavor source 1 for the non-burning inhalation type tobacco product via the inlet opening 51 of the holder 50 passes through the inside of the flavor source 1 for the non-burning inhalation type tobacco product, and is led out from the outlet 3 of the flavor source 1 for the non-burning inhalation type tobacco product without changing the direction. Since the main surface 40b of the heat source 40 does not have air permeability, the air led in from the inlet 2 of the flavor source 1 for the non-burning inhalation type tobacco product is led out from the outlet 3 of the flavor source 1 for the non-burning inhalation type tobacco product without passing through the inside of the heat source 40. That is, the holder 50 has a flow path through which the air led out from the outlet 3 is led to the mouthpiece 59. Specifically, out of the pair of main surfaces 1a and 1b provided on the flavor source 1 for the non-burning inhalation type tobacco product, the flow path is formed along the longitudinal direction L so that the air passes through the inside of the flavor source 1 for the non-burning inhalation type tobacco product. The air led in from the inlet 2 of the flavor source 1 for the non-burning inhalation type tobacco product via the inlet opening 51 of the holder 50 passes through the outlet 3 of the flavor source 1 for the non-burning inhalation type tobacco product, and is led out straight from the mouthpiece 59.

### (4.3.4) Effect

According to the non-burning inhalation type tobacco product 102 of the fourth embodiment, since the flavor source 1 for the non-burning inhalation type tobacco product and the heat source 40 has the plate-shape, as compared to a case in which the flavor source 1 for the non-burning inhalation type tobacco product and the heat source 40 are processed in a columnar shape or a cylindrical shape, it is possible to easily process the flavor source 1 for the non-burning inhalation type tobacco product and the heat source 40. If the heat source 40 is granular in shape, then as compared to a case in which the heat source 40 is processed in a cylindrical shape, particularly, it is possible to easily process the heat source 40.

According to the non-burning inhalation type tobacco product 102 of the fourth embodiment, upon inhalation by the user, the air passes across the entire inside of the flavor source 1 for the non-burning inhalation type tobacco product, because of which it is possible to add a sufficient flavor to the air passing through the flavor source 1 for the non-burning inhalation type tobacco product.

According to the non-burning inhalation type tobacco product 102 of the fourth embodiment, since the air led in from the inlet 2 of the flavor source 1 for the non-burning inhalation type tobacco product passes only through the inside of the flavor source 1 for the non-burning inhalation type tobacco product without passing through the inside of the heat source 40, upon inhalation by the user, it is possible to control the rise in the air-flow resistance.

According to the non-burning inhalation type tobacco product 102 of the fourth embodiment, the size of the non-burning inhalation type tobacco product 102 in the thickness direction T is smaller than the size of the non-burning inhalation type tobacco product 102 in the shorter direction S. Therefore, as compared to a case in which the non-burning inhalation type tobacco product has a columnar shape, it is possible to reduce a size of the non-burning inhalation type tobacco product 102 even if the volume of the non-burning inhalation type tobacco product is the same.

### (5) Examples

Next, an example of a flavor source for the non-burning inhalation type tobacco product will be described.

### (5.1) Test sample

A test sample of the flavor source for the non-burning inhalation type tobacco product was prepared to verify the effect of controlling deposition of menthol component based on the components of the binding agent.

The test sample was prepared using the six types of binding agents shown in Table 6, and six types of test samples according to example 1 through example 4, and comparative example 1 and comparative example 2 were obtained. The grain size of the tobacco material is 0.2 to 0.7 mm, and potassium carbonate is included as an additive. The weight of the tobacco material is 60 mg and the weight of the binding agent is 60 mg. Next, in a state when a mixture of the tobacco material and the binding agent is sandwiched between the nonwoven cloths, thermal fusion bonding was performed at a predetermined temperature from the outer surface of the nonwoven cloth, and the nonwoven cloth and mixed material were formed in the plate-shape as an integrated body.

The temperature of thermal fusion bonding varies depending on the binding agent. Specifically, thermal fusion bonding was performed at 85 to 100°C for EVA, 140°C for PVA, 120°C for polyamide, 120°C for polyurethane, 135°C for polyethylene, and 140°C for polyester. It must be noted that thermal fusion bonding must be performed at a temperature below the heat resistance of the nonwoven fabric. Thus, a binding agent capable of being fused at a relatively low temperature is favorable. For example, since the heat resistance of the nonwoven fabric according to the present example is 140°C, EVA capable of being fused at a relatively low temperature is favorable.

A polypropylene spunbond nonwoven fabric having a basis weight of 15 g/m² is used as the nonwoven fabric. As for the size of a flavor source thus obtained, the length in the longitudinal direction is 45 mm, the length in the shorter direction S is 8 mm, and the length in the thickness direction T is 1 mm. In addition, on the top panel of the above-described flavor source, a 15 mm × 55 mm nonwoven fabric (HOP15H manufactured by Hirose Paper Manufacturing) was made to undergo thermal fusion bonding by aligning the center. In addition, as an exterior body, a 50 mm × 60 mm single-surface laminate paper (basis weight 65 g/m², PE laminate thickness 15 µm) provided with a 4 mm × 35 mm opening was made to undergo thermal fusion bonding with the nonwoven fabric so that the center of the opening was aligned with the center of the flavor source, and by folding the exterior body, a dummy sample of the non-burning inhalation type tobacco product shown in Fig. 5 was prepared, and was provided as the test sample.

### (5.2) Evaluation Method

The amount of addition of menthol component was changed for each test sample, and the deposition state of menthol component under the below-mentioned test conditions was evaluated visually.

As for the test condition, a menthol solution prepared by mixing ethanol, propylene glycol, and menthol component in a ratio of 1:1:6 in the flavor source prepared by the method described above was added in a predetermined quantity, and the amount of addition of menthol component was changed. The amount of addition of the menthol solution is shown in Table 1.

After inserting the test samples one by one in a bag-shaped package made of a 65 mm × 100 mm ethylene vinylalcohol copolymer (EVOH), and storing the package for one week in a temperature controlled bath at 42°C, the samples were retrieved from the temperature controlled bath and stored for three hours in a room at 22°C. Next, the test samples were taken out from the package, and it was visually determined whether or not menthol component was deposited on the surface of the test samples. The deposition state of menthol component was evalulated in three stages, and the effect of controlling deposition on each test sample was evaluated. Moreover, the flavor source of each test sample was extracted by an ethanol solution, and by determining the quantity using a GC/MS (Gas Chromatograph Mass Spectrometer), the ratio of menthol content with respect to the weight of the tobacco material in the flavor source before and after storage was calculated.

Good: No deposition
Fair: Some deposition is seen
Poor: Deposition is seen

The ratio of menthol content with respect to the weight of the tobacco material in the flavor source before and after storage of each test sample, is shown in Table 1, and the evaluation result of the menthol deposition state after the storage test is shown in Table 2.

**[Table 1]**

| | Resin name | Solubility parameter (MPa)^{1/2} | Standard 1 (Amount of addition of solution 20 µL) | | Standard 2 (35 µL) | | Standard 3 (54 µL) | | Standard 4 (73 µL) | |
|---|---|---|---|---|---|---|---|---|---|---|
| | | | Before storage | After storage | Before storage | After storage | Before storage | After storage | Before storage | After storage |
| Example 1 | EVA | 18.6 | - | - | 0.43 | 0.35 | 0.54 | 0.57 | 0.73 | 0.69 |
| Example 2 | Denatured PVA | 19.2 | - | - | 0.44 | 0.30 | 0.53 | 0.50 | - | - |
| Example 3 | Polyamide | 19.4 | - | - | 0.46 | 0.28 | 0.55 | 0.53 | - | - |
| Example 4 | Polyurethane | 20.5 | - | - | 0.44 | 0.30 | 0.56 | 0.54 | - | - |
| Comparative example 1 | PE | 16.4 | 0.21 | 0.17 | 0.42 | 0.28 | - | - | - | - |
| Comparative example 2 | Polyester (denatured PET) | 21.9 | 0.20 | 0.15 | 0.43 | 0.27 | - | - | - | - |

**[Table 2]**

| | Resin name | Solubility parameter (MPₐ)^{1/2} | Standard 1 | Standard 2 | Standard 3 | Standard 4 |
|---|---|---|---|---|---|---|
| Example 1 | EVA | 18.6 | (Good) | Good | Good | Good |
| Example 2 | Denatured PVA | 19.2 | (Good) | Good | Fair | (Poor) |
| Example 3 | Polyamide | 19.4 | (Good) | Good | Fair | (Poor) |
| Example 4 | Polyurethane | 20.5 | (Good) | Good | Fair | (Poor) |
| Comparative example 1 | PE | 16.4 | Poor | Poor | (Poor) | (Poor) |
| Comparative example 2 | Polyester (Denatured PET) | 21.9 | Poor | Poor | (Poor) | (Poor) |

Deposition was seen when the menthol solution was added so that the ratio of the weight of menthol component with respect to the weight of the tobacco material after storage became 0.17 or more and 0.15 or more in the test samples according to the comparative example 1 and the comparative example 2, respectively. The difference between the solubility parameter of the binding agent and the solubility parameter of menthol component in the comparative example 1 is 2.4, and the difference between the solubility parameter of the binding agent and the solubility parameter of menthol component in the comparative example 2 is 3.1. Therefore, it was understood that if the difference between the solubility parameter of the binding agent and the solubility parameter of menthol component is more than 2.4, it becomes difficult to obtain the effect of controlling the deposition of menthol component. Note that since the deposition of menthol component was observed under the condition that the amount of addition of the menthol solution is 35 µL (standard 2), it is obvious that menthol component would be deposited even under the condition when the amount of addition is 54 µL (standard 3) and 73 µL (standard 4), and therefore, the test is not conducted for these standards.

On the other hand, even in a case when the menthol solution was added so that the ratio of the weight of menthol component with respect to the weight of the tobacco material after storage became 0.28 or more in the test sample according to example 1 through example 4, it was understood that the deposition of menthol component could be controlled after storage. Particularly, even in a case when the menthol solution was added so that the ratio of the weight of menthol component with respect to the weight of the tobacco material after storage became 0.69 in the test sample according to example 1, it was understood that the deposition of menthol component could be controlled. In the test sample according to example 1, the solubility parameter of the binding agent is theoretically the closest. Therefore, it is understood that the closer the solubility parameter of the binding agent and the parameter of menthol component, the better the effect of controlling the deposition of menthol component. The test sample according to example 1 is made of EVA, and particularly, it is possible to favorably use EVA as the binding agent. Note that since the deposition of menthol component was not observed under the condition that the amount of addition of the menthol solution is 35 µL (standard 2) in all test samples according to example 1 through example 4, it is obvious that menthol component would not be deposited even under the condition when the amount of addition is 20 µL (standard 1), and therefore, the test is not conducted under the condition when the amount of addition is 20 µL (standard 1). Similarly, since the deposition of menthol component was somewhat observed under the condition that the amount of addition of the menthol solution is 54 µL (standard 3) in the test samples according to example 2 through example 4, it is obvious that menthol component would probably be deposited more significantly under the condition when the amount of addition is 73 µL (standard 4), and therefore, the test is not conducted under the condition when the amount of addition is 73 µL (standard 4) for the test samples according to example 2 through example 4.

As described above, under the precondition that the ratio of the weight of menthol component with respect to the weight of the tobacco material is 0.15 or more, in comparative examples 1 and 2 in which the difference between the solubility parameter of the binding agent and the solubility parameter of menthol component is 2.4 or more, deposition of menthol component was observed, but in examples 1 through 4 in which the difference between the solubility parameter of the binding agent and the solubility parameter of menthol component is 1.7 or less, it was confirmed that the deposition of menthol component was controlled. Based on such experiment results, it was confirmed that the deposition of menthol component is controlled when the difference between the solubility parameter of the binding agent and the solubility parameter of menthol component is 2.0 or less.

### (6) Other embodiments

As described above, the content of the present invention is disclosed through one embodiment according to the present invention. However, it should not be interpreted that the statements and drawings constituting a portion of the present disclosure limit the present invention. From this disclosure, a variety of alternate embodiments will be apparent to one skilled in the art.

In the above-described embodiments, the flavor source 1 for the non-burning inhalation type tobacco product included a nonwoven cloth, but the flavor source 1 for the non-burning inhalation type tobacco product need not necessarily include a nonwoven fabric, and the entire surface of the tobacco compact may be exposed, or a sheet material (for example, paper) other than the nonwoven cloth that covers the tobacco compact may be included.

Moreover, the appearance of the flavor source 1 for the non-burning inhalation type tobacco product is not limited to a rectangular parallelepiped shape, and of course, various other shapes, such as a columnar shape may be included in the present invention.

Thus, it goes without saying that the present invention includes various embodiments and the like not described here. Therefore, the technical range of the present invention is to be defined only by the inventive specific matter according to the adequate claims from the above description.

In addition, the entire content of Japanese Patent Application No. 2012-288532 (filed on December 28, 2012) is incorporated in the present specification by reference.

### [Industrial Applicability]

According to the characteristics of the present invention, the present invention relates to the flavor source for the non-burning inhalation type tobacco product having a granular tobacco material, a binding agent for binding the tobacco material, and menthol component, and also relates to the non-burning inhalation type tobacco product, thus making it possible to provide a flavor source for the non-burning inhalation type tobacco product capable of controlling the deposition of the menthol component.

## Claims

1. A flavor source for a non-burning inhalation type tobacco product formed by thermal fusion bonding, comprising:
a tobacco material having granular shape,
a binding agent that binds the tobacco material, and
a menthol component, wherein
a proportion of a weight of the menthol component with respect to a weight of the tobacco material is 0.15 or more, and
a difference between a solubility parameter of the binding agent and a solubility parameter of menthol component is 2.0 or less.

2. The flavor source for the non-burning inhalation type tobacco product according to claim 1, comprising:
a pair of nonwoven cloths arranged to sandwich the tobacco material and the binding agent, wherein
the flavor source is a plate-shaped formed product having air permeability.

3. The flavor source for the non-burning inhalation type tobacco product according to claim 1 or claim 2, wherein
the binding agent includes any one of agent selected from a group consisting of an ethylene vinylacetate copolymer, a polyvinyl alcohol, polyurethane, polyamide, and a combination of these.

4. The flavor source for the non-burning inhalation type tobacco product according to any one of claim 1 through claim 3, wherein
the binding agent includes an ethylene vinylacetate copolymer.

5. The flavor source for the non-burning inhalation type tobacco product according to any one of claim 1 through claim 4, wherein
a weight of the binding agent with respect to a weight of the tobacco material is 0.5 or more and 3.0 or less.

6. The flavor source for the non-burning inhalation type tobacco product according to any one of claim 1 through claim 5, wherein
the tobacco material includes an additive including at least one of a carbonate or a hydrogen carbonate.

7. A non-burning inhalation type tobacco product, comprising:
the flavor source for the non-burning inhalation type tobacco product according to any one of claim 1 through claim 6:
a holder that contains the flavor source, and
a supporting member that supports the flavor source inside the holder and increases an exposed area of the flavor source within the holder beyond a cross-sectional area specified by the inner circumference of the holder.

8. The non-burning inhalation type tobacco product according to claim 7, wherein
the holder has a longitudinal axial line, a front end, and a rear end as a mouthpiece end, and defines an air flow path leading an external air entered from a front end side up to a mouthpiece end, and
the supporting member supports the flavor source inside the holder, partitions the air flow path into an upstream portion provided on the front end side and a downstream portion provided on a mouthpiece end side using the flavor source, and increases the exposed area of the flavor source that faces at least either one of the upstream portion and the downstream portion beyond the cross-sectional area specified by the inner circumference of the holder.

9. The non-burning inhalation type tobacco product according to claim 7, wherein
the holder is a tubular member formed by stacking at least three sheets having flexibility, and
the tubular member comprises:
a first air flow path that opens at one end of the tubular member and is formed between a central sheet positioned in a center, and a first sheet which is one of remaining sheets;
a second air flow path that opens at the other end of the tubular member and is formed between the central sheet and a second sheet which is one of the remaining sheets other than the first sheet; and
an aperture that communicates the first and second air flow paths and is formed on the central sheet, wherein
the flavor source is arranged to cover the aperture on the central sheet.

10. A non-burning inhalation type tobacco product comprising:
the flavor source for the non-burning inhalation type tobacco product according to any one of claim 1 through claim 6:
a heat source that heats the flavor source without burning, and having a plate-shape including a pair of main surfaces, and
a holder that contains the heat source and the flavor source, wherein
the flavor source has a plate-shape including a pair of main surfaces, and
the heat source and the flavor source are laminated inside the holder so that one of the main surfaces provided on the heat source and one of the main surfaces provided on the flavor source are facing each other,
the flavor source has an inlet that leads air into the flavor source, and an outlet that leads the air out from the flavor source, and
the inlet and the outlet are provided on the surfaces other than the main surface facing the heat source among surfaces provided on the flavor source.

11. A non-burning inhalation type tobacco product, comprising:
the flavor source for the non-burning inhalation type tobacco product according to any one of claim 1 through claim 6;
a heat source that heats the flavor source; and
a holder that contains the flavor source and the heat source, wherein
the heat source includes a latent heat storage material.

## Patentansprüche

1. Aromaquelle für ein Tabakprodukt vom nicht brennenden Inhalationstyp, das durch Wärmefusionsbindung gebildet wird, umfassend:
ein Tabakmaterial mit einer Granulatform,
ein Bindemittel, welches das Tabakmaterial bindet, und
einen Mentholbestandteil, wobei
ein Gewichtsanteil des Mentholbestandteils bezüglich eines Gewichtes des Tabakmaterials 0,15 oder mehr ist, und
ein Unterschied zwischen einem Löslichkeitsparameter des Bindemittels und einem Löslichkeitsparameter des Mentholbestandteils 2,0 oder weniger ist.

2. Aromaquelle für das Tabakprodukt vom nicht brennenden Inhalationstyp nach Anspruch 1, umfassend:
zwei Vliestücher, die derart angeordnet sind, dass das Tabakmaterial und das Bindemittel dazwischen liegen, wobei
die Aromaquelle ein plattenförmiges Produkt mit Luftdurchlässigkeit ist.

3. Aromaquelle für das Tabakprodukt vom nicht brennenden Inhalationstyp nach Anspruch 1 oder Anspruch 2, wobei das Bindemittel eines der Mittel enthält, die ausgewählt sind aus der Gruppe, bestehend aus einem Ethylenvinylacetatcopolymer, einem Polyvinylalkohol, Polyurethan, Polyamid, und einer Kombination von diesen.

4. Aromaquelle für das Tabakprodukt vom nicht brennenden Inhalationstyp nach einem der Ansprüche 1 bis 3, wobei das Bindemittel ein Ethylenvinylacetatcopolymer enthält.

5. Aromaquelle für das Tabakprodukt vom nicht brennenden Inhalationstyp nach einem der Ansprüche 1 bis 4, wobei ein Gewicht des Bindemittels bezüglich eines Gewichtes des Tabakmaterials 0,5 oder mehr und 3,0 oder weniger beträgt.

6. Aromaquelle für das Tabakprodukt vom nicht brennenden Inhalationstyp
nach einem der Ansprüche 1 bis 5, wobei
das Tabakmaterial ein Additiv enthält, das mindestens eines aus einem Carbonat und einem Wasserstoffcarbonat enthält.

7. Tabakprodukt vom nicht brennenden Inhalationstyp, umfassend:
die Aromaquelle für das Tabakprodukt vom nicht brennenden Inhalationstyp nach einem der Ansprüche 1 bis 6,
ein Halteelement, das die Aromaquelle umfasst, und
ein Unterstützungselement, das die Aromaquelle in dem Halteelement unterstützt und einen exponierten Bereich der Aromaquelle innerhalb des Halteelementes über einen Querschnittsbereich hinaus erhöht, der durch den inneren Umfang des Halteelementes bestimmt ist.

8. Tabakprodukt vom nicht brennenden Inhalationstyp nach Anspruch 7, wobei
das Halteelement eine längsgerichtete Axiallinie, ein vorderes Ende und ein hinteres Ende als ein Mundstückende aufweist und einen Luftstromweg definiert, der eine Luft von außen, die von einer Vorderendenseite hin zu einem Mundstückende führt, und
das Unterstützungselement die Aromaquelle innerhalb des Halteelementes unterstützt, den Luftstromweg in einen stromaufwärtsgelegenen Abschnitt, der an der Vorderendenseite zur Verfügung gestellt ist, und einen stromabwärtsgelegenen Abschnitt, der an einer Mundstückendenseite zur Verfügung gestellt ist, teilt, wobei die Aromaquelle verwendet wird, und den exponierten Bereich der Aromaquelle, der mindestens einem des stromaufwärtsgelegenen Abschnitts oder des stromabwärtsgelegenen Abschnitts gegenüber liegt, über den Querschnittsbereich hinaus, der durch den Innenumfang des Halteelementes spezifiziert ist, erhöht.

9. Tabakprodukt vom nicht brennenden Inhalationstyp nach Anspruch 7, wobei
das Halteelement ein rohrförmiges Element ist, das gebildet wird, indem mindestens drei flexible Lagen übereinander gestapelt werden, und
wobei das rohrförmige Element Folgendes umfasst:
einen ersten Luftstromweg, der sich an einem Ende des rohrförmigen Elementes öffnet und zwischen einer mittigen Lage, die in einer Mitte positioniert ist, und einer ersten Lage, die eine der verbleibenden Lagen ist, gebildet wird;
einen zweiten Luftstromweg, der sich zum anderen Ende des rohrförmigen Elementes öffnet und zwischen der Mittellage und der zweiten Lage, die eine der verbleibenden Lagen und nicht die erste Lage ist, gebildet ist; und
eine Öffnung, die den ersten und den zweiten Luftstromweg miteinander verbindet und auf der Mittellage gebildet ist, wobei
die Aromaquelle derart angeordnet ist, dass sie die Öffnung auf der Mittellage bedeckt.

10. Tabakprodukt vom nicht brennenden Inhalationstyp, umfassend:
die Aromaquelle für das Tabakprodukt vom nicht brennenden Inhalationstyp nach einem der Ansprüche 1 bis 6;
eine Wärmequelle, welche die Aromaquelle erwärmt, ohne zu brennen, und mit einer Plattenform, die zwei Hauptoberflächen umfasst, und
ein Halteelement, das die Wärmequelle und die Aromaquelle umfasst, wobei
die Aromaquelle eine Plattenform aufweist, die zwei Hauptoberflächen umfasst, und
die Wärmequelle und die Aromaquelle in dem Halteelement laminiert sind, derart, dass sich eine der Hauptoberflächen, die auf der Wärmequelle zur Verfügung gestellt ist, und eine der Hauptoberflächen, die auf der Aromaquelle zur Verfügung gestellt ist, gegenüberliegen;
die Aromaquelle einen Einlass aufweist, der Luft in die Aromaquelle hinein lässt, und einen Auslass, der die Luft aus der Aromaquelle hinaus lässt, und
der Einlass und der Auslass auf den Oberflächen zur Verfügung gestellt sind, die andere als die Hauptoberfläche sind, die der Wärmequelle gegenüber liegen, aus Oberflächen, die auf der Aromaquelle zur Verfügung gestellt sind.

11. Tabakprodukt vom nicht brennenden Inhalationstyp, umfassend:
die Aromaquelle für das Tabakprodukt vom nicht brennenden Inhalationstyp nach einem der Ansprüche 1 bis 6;
eine Wärmequelle, welche die Aromaquelle erwärmt; und
eine Haltevorrichtung, welche die Aromaquelle umfasst und die Wärmequelle umfasst, wobei
die Wärmequelle ein latent wärmespeicherndes Material enthält.

## Revendications

1. Source aromatique destinée à un produit de tabac de type à inhalation sans combustion, formée par liaison par fusion à chaud, comprenant :
une matière de tabac présentant une forme granulaire,
un liant qui lie la matière de tabac, et
un composant menthol, dans lequel :
une proportion de poids du composant menthol par rapport à un poids de la matière de tabac est supérieure ou égale à 0,15, et
une différence entre un paramètre de solubilité du liant et un paramètre de solubilité du composant menthol est inférieure ou égale à 2,0.

2. Source aromatique destinée au produit de tabac de type à inhalation sans combustion, formée selon la revendication 1, comprenant :
une paire de tissus disposés pour prendre en sandwich la matière de tabac et le liant, dans laquelle :
la source aromatique est un produit façonné en forme de plaque présentant une perméabilité à l'air.

3. Source aromatique destinée au produit de tabac de type à inhalation sans combustion, selon la revendication 1 ou la revendication 2, dans laquelle :
le liant inclut un agent quelconque choisi dans un groupe constitué d'un copolymère d'éthylène-vinylacétate, d'un poly(alcool) de vinyle, du polyuréthane, du polyamide, et une d'une combinaison de ceux-ci.

4. Source aromatique destinée au produit de tabac de type à inhalation sans combustion, selon l'une quelconque des revendications 1 à 3, dans laquelle :
le liant inclut un copolymère d'éthylène-vinylacétate.

5. Source aromatique destinée au produit de tabac de type à inhalation sans combustion, selon l'une quelconque des revendications 1 à 4, dans laquelle :
un poids du liant par rapport à un poids de la matière de tabac est supérieur ou égal à 0,5 et inférieur ou égal à 3,0.

6. Source aromatique destinée au produit de tabac de type à inhalation sans combustion, selon l'une quelconque des revendications 1 à 5, dans laquelle :
la matière de tabac inclut un additif incluant un carbonate et/ou un hydrogénocarbonate.

7. Produit de tabac de type à inhalation sans combustion, comprenant :
la source aromatique destinée au produit de tabac de type à inhalation sans combustion selon l'une quelconque des revendications 1 à 6 ;
un organe de maintien qui contient la source aromatique, et
un élément de support qui supporte la source aromatique à l'intérieur de l'organe de maintien et accroît une aire exposée de la source aromatique à l'intérieur de l'organe de maintien au-delà de l'aire de section transversale spécifiée par la circonférence intérieure de l'organe de maintien.

8. Produit de tabac de type à inhalation sans combustion selon la revendication 7, dans lequel :
l'organe de maintien présente une ligne longitudinale axiale, une extrémité avant et une extrémité arrière en tant qu'extrémité d'embout, et définit un trajet d'écoulement d'air conduisant de l'air extérieur ayant pénétré par un côté d'extrémité avant jusqu'à une extrémité d'embout, et
l'élément de support supporte la source aromatique à l'intérieur de l'organe de maintien, sépare le trajet d'écoulement d'air en une partie amont se trouvant du côté d'extrémité avant et en une partie aval se trouvant d'un côté d'extrémité d'embout, à l'aide de la source aromatique, et accroît l'aire exposée de la source aromatique qui est en regard de l'une et/ou l'autre de la partie amont et de la partie aval au-delà de l'aire de section transversale spécifiée par la circonférence intérieure de l'organe de maintien.

9. Produit de tabac de type à inhalation sans combustion selon la revendication 7, dans lequel :
l'organe de maintien est un élément tubulaire formé par empilage d'au moins trois feuilles présentant une flexibilité, et
l'élément tubulaire comprend :
un premier trajet d'écoulement d'air qui aboutit à une extrémité de l'élément tubulaire et qui est formé entre une feuille centrale positionnée en un centre, et une première feuille qui est l'une des feuilles restantes ;
un second trajet d'écoulement d'air qui aboutit à l'autre extrémité de l'élément tubulaire et qui est formé entre la feuille centrale et une seconde feuille qui est l'une des feuilles restantes, autre que la première feuille ; et
une ouverture qui fait communiquer les premier et second trajets d'écoulement d'air et qui est ménagée sur la feuille centrale, dans lequel :
la source aromatique est disposée pour recouvrir l'ouverture sur la feuille centrale.

10. Produit de tabac de type à inhalation sans combustion, comprenant :
la source aromatique destinée au produit de tabac de type à inhalation sans combustion selon l'une quelconque des revendications 1 à 6 ;
une source de chaleur qui chauffe la source aromatique sans combustion, et ayant une forme de plaque présentant une paire de surfaces principales, et
un organe de maintien qui contient la source de chaleur et la source aromatique, dans lequel :
la source aromatique a une forme de plaque présentant une paire de surfaces principales, et
la source de chaleur et la source aromatique sont stratifiées à l'intérieur de l'organe de maintien, de manière que l'une des surfaces principales se trouvant sur la source de chaleur et l'une des surfaces principales se trouvant sur la source aromatique soient en regard l'une de l'autre,
la source aromatique comporte une entrée qui conduit l'air dans la source aromatique, et une sortie qui conduit l'air hors de la source aromatique, et
l'entrée et la sortie se trouvent sur les surfaces autres que la surface principale en regard de la source de chaleur parmi les surfaces se trouvant sur la source aromatique.

11. Produit de tabac de type à inhalation sans combustion, comprenant :
la source aromatique destinée au produit de tabac de type à inhalation sans combustion selon l'une quelconque des revendications 1 à 6 ;
une source de chaleur qui chauffe la source aromatique ; et
un organe de maintien qui contient la source aromatique et la source de chaleur, dans lequel :
la source chaleur inclut une matière d'accumulation de chaleur latente.
